# EUROPEAN PATENT SPECIFICATION

(11) **EP 0 642 774 B1**
(45) Date of publication and mention of the grant of the patent: **23.08.2000**
(21) Application number: 93307108.6
(22) Date of filing: 09.09.1993
(51) Int. Cl.: A61F 2/30

(54) **Discontinuous fibre composite implant prosthesis**
Endoprothese aus Verbundstoff mit diskontinuierlichen Fasern
Prothèse implantable composite à fibres discontinues

(43) Date of publication of application: 15.03.1995
(73) Proprietor: Shirandami, Roozbeh, Dr., London SE19 2LH (GB)
(72) Inventor: Roozbeh, Shirandami, Dr., Upper Norwood, London SE19 2LH (GB); Ilhan, Esat Ibrahim, Dr., Cockfosters, London EN4 0BQ (GB)

(56) References cited:
- EP-A- 0 025 835
- EP-A- 0 171 884
- WO-A-87/00033
- WO-A-87/04916
- WO-A-93/19699
- US-A- 3 893 196

## Description

This invention relates to the form and structure of a composite implant prosthesis.

Heretofore, patents WO-A-87-04961, EP-171884 and US-A-3-893-196 disclose composite implant prosthesis comprising a core of continuous fibers extending over the entire length of prosthesis formed by moulding and machining, over layed by a layer of transversely oriented continuous fibres and additional layer of polymer to allow disposition adjacent to bone.

The most relevant prior art is described in document WO-A-93 19699, which document falls under the provisions according to Art. 54(3) EPC. There is disclosed composite implant comprising an elongated core formed of short filament fibres having a length of less than four millimetres embedded in thermoplastic polymer; and encasing the core, a sheath formed of elongated carbon filament fibres embedded in a thermoplastic polymer wound around the core in layers and moulded thereto.

US patent no. 4,902,297 describes the above mentioned machining of core as detrimental. It discloses a composite implant prosthesis formed by a plurality of substantially unidirectional strands of fibre, each fibre extending substantially over the complete length of the core, an inner casing defined by braided material enclosing the core, and an outer casing enclosing the inner casing. The core and the inner casing defining a substantially uniform width from one end to the other end, while the outer casing defines an irregular width from one end to the other to accommodate disposition of the prosthesis adjacent to a bone. Here the core is subjected to a substantial portion of the load applied to the composite hip prosthesis. Since the dimension of the core and first casing are substantially constant along the implant length from one end to the other, variation in the stiffness along the length of implant can not be accommodated with this design. This shortcoming is due to the manufacturing process employed in the making of the core and first casing. Here the core and first casing are made into a single straight rod of uniform width using pultrusion. This rod is then placed into a forming press where heat and pressure are used to form or bend the rod to the shape of the implant.

With the present invention it is possible to form a composite implant prosthesis comprises of a core and first casing which can vary in width from one end to the other. This allows the width of the core and first casing to vary along the implant length to achieve variation in the implant stiffness along the implant length from one end to the other and to define an overall irregular width to accommodate disposition of the prosthesis adjacent to a bone. These sections may, therefore, in one embodiment of the invention, be produced in the shape of the final profile of the implant producing a complete implant and, therefore, require no forming process to shape the core and first casing.

The invention comprises a composite implant prosthesis with a core formed by reinforcement of a matrix using discontinuous fibres aligned predominantly unidirectional along the implant length, and a first casing enclosing the core, with discontinuous fibres predominantly orientated transversely to that of the core, to form a circumferentially reinforced first casing.

A method for constructing the composite implant includes the steps of:
a) forming a core of unidirectionally aligned discontinuous fibre reinforcement along the implant, using a closed moulding process.
b) using the section produced in (a) as an insert in a second closed moulding process to allow over moulding of the core in (a), and hence producing the first casing encapsulating the core. The orientation of the fibre reinforcement in this casing will be predominantly transverse to that of the core, to produce circumferential reinforcement.

The described manufacturing process allows the cross section of the unidirectional aligned core and circumferentially reinforced first casing to vary along the implant length as well as having different proportions of reinforcement. The overall width of the composite implant prosthesis can, therefore, be varied along the prosthesis length from one end to the other to accommodate disposition of the prosthesis adjacent to a bone.

An alternative method of production is to manufacture the core and the first casing separately and then bond them together.

Embodiments of the invention will now be described by way of example with reference to the accompanying drawings in which:
- Fig. 1: is a side view of one embodiment of composite implant prosthesis according to the invention;
- Fig. 2: is a side view of another embodiment of a composite implant prosthesis having a collar;
- Fig. 3: is a cross section view taken along line A-A of Figs 1 and 2 of the composite implant prosthesis (without the out sleeve 3).
- Fig. 4: is a schematic illustration of the fibre orientation and elastic moduli along the implant prosthesis (without the outer sleeve 3);
- Fig. 5: is a schematic illustration of a re-entrant socket into a ball joint which may be provided on the implant prosthesis; and
- Fig. 6: is a schematic illustration of an alternative form of ball joint which is included in the closed moulding of the core 1 and first casing 2 to product a complete implant prosthesis.

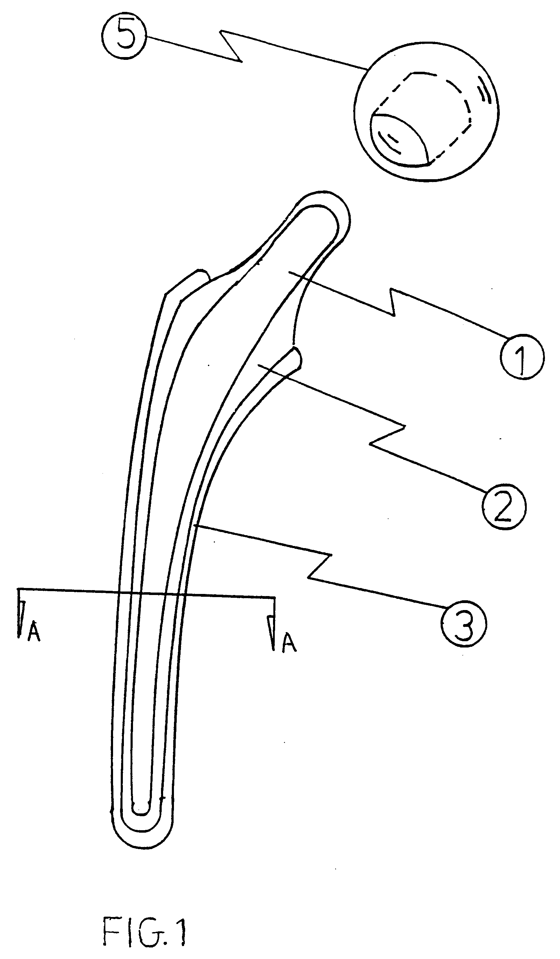

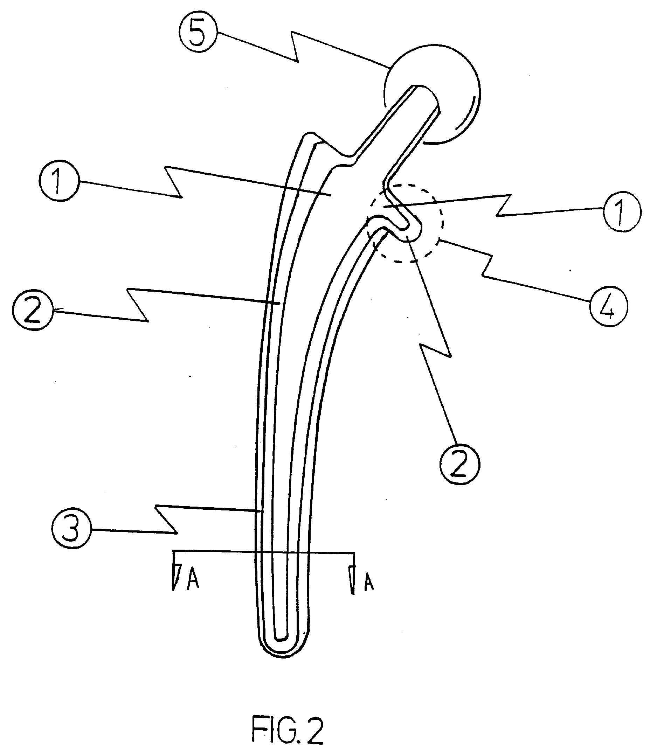

Turning to Figures 1 and 2 , the composite hip prosthesis constructed in accordance with the process described above, includes a central core 1, and a first casing 2. In the case of the collared composite hip prosthesis shown in Fig. 2, a sandwich structure is formed by extending the core (1) and first casing (2) into the collar (4). The stiffness of this sandwich structure is optimised by controlling the thickness of the core (1) and first casing (2) to optimise the load on the calcar femoral.

To accommodate the variation in the femoral canal shape from patient to patient, a separate outer sleeve, (3) may be formed by a closed moulding process. This is bonded on to the prothesis stem (as shown in Figures 1 and 2) during or prior to surgery. The outer sleeve preferably comprises material with a porous surface to achieve fixation of the prosthesis by means of mechanical interlocking achieved through bone ingrowth.

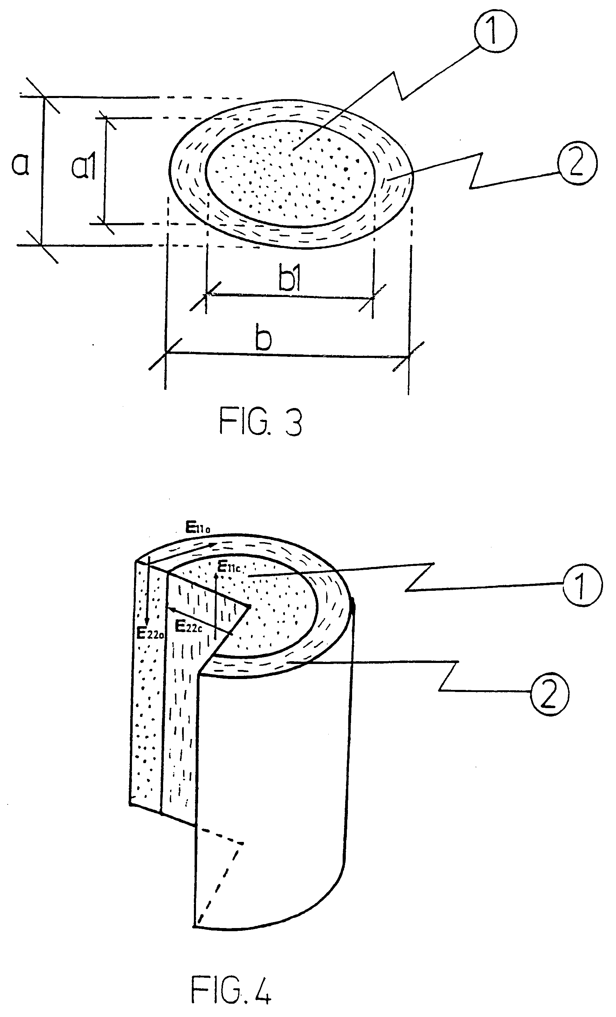

In a preferred embodiment, the radial dimensions of the core and the first casing as shown in Fig. 3, are such that a ≥ a1 and b ≥b1. The dimensions of the core (1) and the first casing (2) as shown in Fig. 3 are varied along the length of the implant to optimise the stiffness of the implant along its length and to control the overall width of the implant to accommodate disposition of the prosthesis adjacent to a bone.

The arrangement of the reinforcement as designed in the present invention leads to an implant with layered stiffness. The stiffness in the longitudinal direction to the stem is reduced across the cross section of the implant, going from the longitudinally aligned core (1) to the transversely aligned first casing (2). Conversely the stiffness in the transverse direction to the stem is increased across the cross section of the implant, going from the aligned core (1) to the first casing (2). In a preferred embodiment, the Young's moduli, E, for the core (1) and first casing (2) shown in Fig. 4 will be such that E11c ≥ E11o and E22c ≥ E22o.

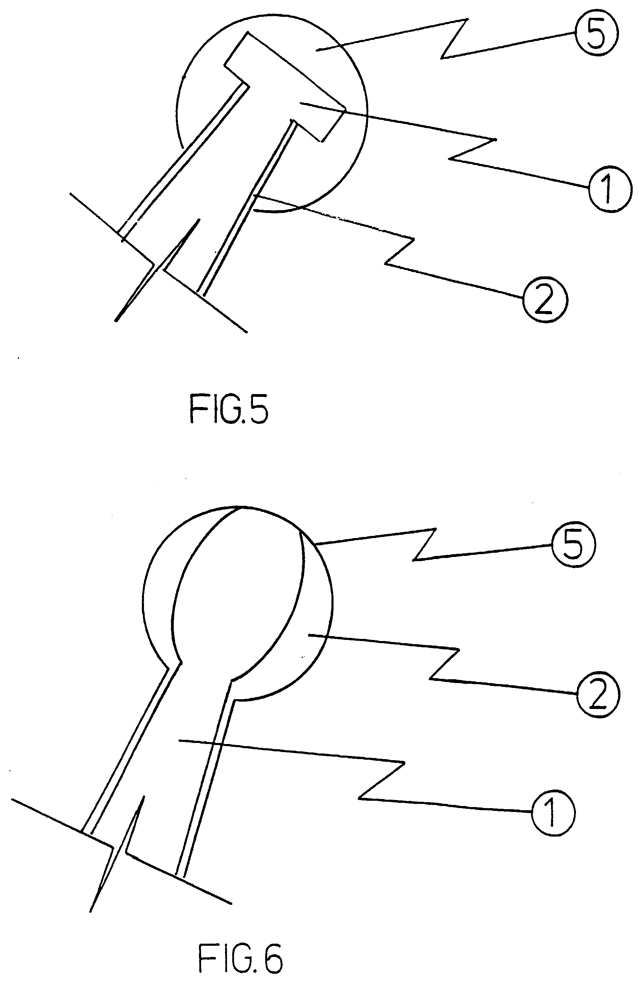

The ball joint of the prosthesis (5) is manufactured using a separate closed moulding process. This can be attached to the implant neck either by bonding or by a form of re-entrant socket into the ball to form a mechanical fixation as shown in Fig. 5.

Alternatively, as shown in Fig. 6, the ball joint (5) can be included in the closed moulding process of the core (10) and first casing (2) to produce a complete prosthetic implant.

## Claims

1. A composite implant prosthesis comprising a core (1) having a matrix and a first casing (2) wherein:
the core (1) formed by reinforcement of the matrix using discontinuous fibres aligned predominantly unidirectionally along the implant length, and the first casing (2) enclosing the core (1), with discontinuous fibres predominantly oriented transversely to that of the core (1) to form a circumferentially reinforced first casing (2).

2. A prosthesis according to Claim 1 wherein variation in the stiffness of the composite implant prosthesis along its length, is controlled by means of varying the dimensions of the aligned core (1) and first casing (2) along the length of the implant.

3. A prosthesis according to Claim 1 or 2 wherein the dimensions of the aligned core (1) and first casing (2) we varied to define an overall irregular width to accommodate disposition of the prosthesis adjacent to a bone.

4. A prosthesis according to Claim 1, 2 or 3 wherein the core (1) and first casing (2) extend to form a structural collar (4) with a sandwich structure the stiffness of which is optimised by controlling the thickness of the core (1) and first casing (2) forming the sandwich structure at the collar.

5. A prosthesis according to any preceding claim provided with a ball joint (5) moulded with the core (1) and first casing (2) to produce a complete implant.

6. A prosthesis according to any of Claims 1 to 4 provided with a ball joint (5) attached thereto by bonding or by a re-entrant socket in the ball joint (5).

7. A prosthesis according to any preceding claims wherein an outer sleeve (3) is provided to correct the shape of the prosthesis so as to accommodate variation in femoral canal shape form patient to patient.

8. A prosthesis according to Claim 7 wherein the outer sleeve (3) comprises material with a porous surface to achieve fixation of the prosthesis by means of mechanical interlocking achieved through bone ingrowth.

## Patentansprüche

1. Eine Verbundprothese, die aus einem Kern besteht (1) und eine Matrix und ein erstes Gehäuse (2) hat, worin: der Kern (1) durch Verstärkung der Matrix gebildet wird, welche unterbrochenen Fasern verwendet, die überwiegend unidirectional entlang ausgerichtet werden, und das erste Gehäuse (2), das den Kern (1), mit unterbrochenen Fasern überwiegend quer zum Kern orientierten Fasern umgibt (1), um ein umfangsmäßig verstärktes erstes Gehäuse (2) zu bilden.

2. Eine Prothese entsprechend Anspruch 1, worin eine Veränderung der Steifheit der Verbundprothese entlang seiner Länge mittels des Veränderns der Maße des ausgerichteten Kernes (1) und erstes Gehäuse (2) entlang der Länge kontrolliert wird.

3. Eine Prothese entsprechend Anspruch 1 oder 2, worin die Maße des ausgerichteten Kernes (1) und des ersten Gehäuses (2) verändert werden, um eine gesamte unregelmäßige Breite zu definieren, um die Einteilung der Prothese neben einem Knochen anzupassen.

4. Eine Prothese entsprechend Anspruch 1, 2 oder 3, worin der Kern (1) und das erste Gehäuse (2) sich ausdehnen, um einen strukturellen Stellring (4) mit einer Sandwichstruktur zu bilden, dessen Steifigkeit optimiert wird, indem man die Stärke des Kernes (1) und des ersten Gehäuses (2), welches die Sandwichstruktur am Stellring bildet steuert.

5. Eine Prothese entsprechend jeder vorausgegangenen Forderung versehen mit einem Kugelgelenk (5), das mit dem Kern (1) geformt wurde und das erste Gehäuse (2) um eine komplette Prothese zu produzieren.

6. Eine Prothese entsprechend irgendwelchen von Ansprüchen 1 bis 4 versehen mit einem Kugelgelenk (5), das durch Verkleben oder durch eine einspringende Einfaßung im Kugelgelenk (5) angebracht wird.

7. Eine Prothese entsprechend den vorausgegangenen Forderungen, worin eine äußere Hülse (3) zur Verfügung gestellt wird, um die Form der Prothese zu korregieren, um Veränderung des femoral Kanalform-Formularpatienten zum Patienten anzupassen.

8. Eine Prothese entsprechend Anspruch 7, worin die äußere Hülse (3) Material mit einer porösen Oberfläche enthält, um Fixierung der Prothese mittels des mechanischen Ineinandergreifens zu erzielen, das durch Knochenverwachsung erzielt wird.

## Revendications

1. Un composé implante la prothèse comportant un noyau (1) ayant une matrice et une première enveloppe (2) où: le noyau (1) a formé par le renfort de la matrice utilisant les fibres discontinues alignées principalement unidirectionally le long du implantent la longueur, et la première enveloppe (2) entourant le noyau (1), avec les fibres discontinues principalement orientées transversalement à celle du noyau (1) pour former une première enveloppe circonférentiellement renforcée (2).

2. Une prothèse selon la revendication 1 où la variation de la rigidité du composé implantent la prothèse sur sa longueur, est commandée au moyen de changer les dimensions du noyau aligné (1) et la première enveloppe (2) sur la longueur du implantent.

3. Une prothèse selon la revendication 1 ou 2 où les dimensions du noyau aligné (1) et de la première enveloppe (2) sont changées pour définir une largeur irrégulière globale pour faciliter la destination de la prothèse à côté d'un os.

4. Une prothèse selon la revendication 1, 2 ou 3 où le noyau (1) et la première enveloppe (2) étendent pour former un collier structural (4) avec une structure de sandwich dont la rigidité est optimalisée en contrôlant l'épaisseur du noyau (1) et de la première enveloppe (2) formant la structure de sandwich au collier.

5. Une prothèse selon une revendication précédente quelconque a fourni en joint à rotule (5) moulé avec le noyau (1) et la première enveloppe (2) pour produire un complet implantent.

6. Une prothèse selon n'importe laquelle de revendications 1 à 4 a fourni en joint à rotule (5) attaché là-dessus en collant ou par un plot de réentrée dans le joint à rotule (5).

7. Une prothèse selon revendications précédentes quelconques où une douille externe (3) est fournie pour corriger la forme de la prothèse afin de faciliter la variation du patient fémoral de forme de forme de canal au patient.

8. Une prothèse selon la revendication 7 où la douille externe (3) comporte le matériel avec une surface poreuse pour réaliser la fixation de la prothèse au moyen d'enclencher mécanique réalisé par l'ingrowth d'os.
